# EUROPEAN PATENT APPLICATION

(11) **EP 3 257 485 A1**
(43) Date of publication of application: **20.12.2017**
(21) Application number: 16175058.3
(22) Date of filing: 17.06.2016
(51) Int. Cl.: A61F 7/00

(54) **APPLICATOR FOR A REFRIGERANT**

(71) Applicant: Trimb Healthcare AB, 114 26 Stockholm (SE)
(72) Inventor: SOPKO, Martin Roland, 115 22 Stockholm (SE); WITTE, Johannes Hendricus, 2111 BN Aerdenhout (NL)
(74) Representative: De Vries & Metman

(57) **Abstract**

Applicator, such as a roller applicator, comprising a cooling element, such as a metal ball, and at least two flow channels extending between respective refrigerant inlets and refrigerant outlets. The flow channels provide a different thermal conductivity between the cooling element and a refrigerant passing the respective flow channel. The applicator comprises at least one stop for selectively blocking one or more of the flow channels, to adjust the overall heat exchange between the refrigerant and the cooling element.

## Description

The present invention relates to an applicator having a cooling element with a cooling surface and flow channels for guiding a refrigerant along the cooling element. Such applicators may for instance be used for locally cooling and massaging a user's skin, e.g., during application of a cosmetic or medical compound.

It is an object of the invention to provide an applicator providing a cooling effect that more accurately matches a desired temperature for a specific application or a specific user.

The object of the invention is achieved with an applicator comprising a cooling element, at least two flow channels extending between a refrigerant inlet and a refrigerant outlet, the flow channels providing different thermal conductivity between the cooling element and a refrigerant passing the respective flow channel. The applicator comprises a stop for selectively blocking one or more of the flow channels.

These measures allow adjustment of the amount of heat that the refrigerant can extract from the cooling element, so the cooling effect can be regulated. In one embodiment, an end user may be able to actively adjust the cooling effect. In another possible embodiment a manufacturer may make a selection of flow channels to adjust the cooling effect for a given active compound and fixate it.

The thermal conductivity provided by the flow channels can be differentiated, e.g., by varying the length and/or width of the channels so the cooling effect is adjusted by varying the contact surface between the refrigerant and the cooling element. Alternatively, some of the channels may provide direct contact between the refrigerant and the cooling element, while other channels may provide indirect thermally conductive interaction between the refrigerant and the cooling element, e.g., via a shell containing the cooling element.

In an exemplary embodiment, the flow channels may comprise:
- one or more direct heat exchange channels confined by the surface of the cooling element;
- one or more indirect heat exchange channels separated from the surface of the cooling element. The one or more stops can selectively be positioned into a first position closing at least some of the direct heat exchange channels and opening at least some of the indirect heat exchange channels, or into a second position closing at least some of the indirect heat exchange channels and opening at least some of the direct heat exchange channels. The indirect heat exchange channels may for example be separated from the cooling element by the afore mentioned shell holding the cooling element.

The applicator may comprise a supply channel communicating with a reservoir for a refrigerant. The one or more stops can selectively be positioned into a first position closing the direct heat exchange channels and connecting the indirect heat exchange channels to the supply channel, or into a second position closing the indirect heat exchange channels and connecting the direct heat exchange channels to the supply channel.

In a specific embodiment, the cooling element may be mounted in a shell and protrude out of the shell to present a contact surface, e.g., for contacting the skin of a user. The flow channels may be provided as recesses in the shell. For instance, the direct heat exchange channels may be formed as recesses at the surface of the shell facing the cooling element, while the indirect heat exchange channels are formed by recesses at an opposite surface of the shell.

The shell can for example be held in a seat. The relative position of the shell and the seat can be adjustable. For example, the shell can rotate relative to the seat and/or the seat can rotate relative to the shell.

Optionally, the stop may comprise at least one projection of the seat. If the number of projections, the number of direct heat exchange channels and the number of indirect heat exchange channels are equal, the direct and indirect heat exchange channels can each be provided with a recess matching the projections, so as to receive the projection in a manner blocking the respective channels. If the projections are received in respective recesses in the direct heat exchange channels in said first position, the direct heat exchange channels will be blocked. If the projections are received in respective recesses in the indirect heat exchange channels in said second position, the indirect heat exchange channels will be blocked.

The applicator can for example be a roller applicator with a rotatable spherical cooling element. During massaging the spherical cooling element rotates in the shell and can for example dispense an active ingredient. The contact surface cools the user's skin. The contact surface is not a fixed part of the cooling element but changes with the rotation of the cooling element.

Surprisingly, it has been found that with such a roller applicator, held in a shell supported by a seat, a mixture of a refrigerant with an active ingredient is not sprayed but rather spreads itself over the contact surface, thus enabling effective dispensing via the cooled roller ball.

If the cooling element is spherical, each channel may for example extend over the surface of the cooling element in a plane through a longitudinal axis of the applicator. Alternatively, they may make an angle with the longitudinal axis, e.g., in a spiralling manner.

The seat, the cooling element and the optional shell may be held in a casing. The casing may be a cylindrical casing with an open top end exposing the contact surface of the cooling element. The open top end may have a diameter smaller than the diameter of the cooling element and the optional shell. For example, the casing may narrow down in the direction of the open top end with a curvature matching the radius of the cooling element or the radius of the optional shell. The shell may be spherical with an open top end exposing the contact face of the cooling element. The open top end of the shell may for example be at substantially the same level as the open top end of the casing.

The casing and the seat may be connected by matching profiles. For example, the casing may be provided with an annular recess at its inner surface receiving an annular rim at an outer surface of the seat. Alternatively, the seat may be provided with an annular recess at its outer surface receiving an annular rim at an inner surface of the casing.

Optionally, the seat may be provided with a cylindrical passage dimensioned to receive a nozzle of an aerosol container, e.g., a depressible nozzle releasing pressurized content when it is pushed down. In that case the annular recess at the inner surface of the casing may have a width larger than the width of the received rim of the seat. This allows movement of the seat in a longitudinal direction of the applicator. In use, the depressible nozzle will resiliently push the seat and the cooling element upwardly against the narrowing edge of the casing's open top end. When a user massages his skin, the applied pressure will push the cooling element and the seat inwardly and actuate the nozzle of the aerosol container. As a result, the nozzle releases liquefied refrigerant from the aerosol container which will flow through the open channels. Due to the pressure drop the refrigerant will expand and evaporate during flowing through the channels. This expansion extracts heat from the cooling element, which cools down. An active component can be released jointly with the refrigerant, e.g., as a mixture, and can be applied on the user's skin using the cooled contact face. The effectiveness of the active compound can substantially be improved by the combined cooling and massaging action.

The cylindrical casing may have a lower edge profiled to form a snap joint with an upper collar of an aerosol container.

While the cooling element is typically made of a material of a high thermal conductivity, such as stainless steel, the partition separating the second flow channel from the cooling element is of a lower thermal conductivity. The cooling element may for example have a heat conductivity of at least 10 W⁻¹.m⁻¹.K⁻¹, e.g., at least 15 W⁻¹.m⁻¹.K⁻¹. The partition may for example have a heat conductivity of at least 0,02 W⁻¹.m⁻¹.K⁻¹, e.g., at least 0,5 W⁻¹.m⁻¹.K⁻¹. For instance, the cooling element can be made of a metal, e.g., stainless steel or an aluminium type. The partition can, e.g., be polymeric, e.g., of acrylonitril-butadieen-styreen (ABS), polypropylene or polyethylene.
The invention also relates to an applicator comprising a single reservoir for containing a mixture of a liquid refrigerant and a cosmetically and/or pharmaceutically active component. The active component is applied together with the refrigerant via the contact face of the cooling element.

Refrigerant is to mean any liquid or gas which is able to extract heat due to evaporation and/or expansion or other cooling processes. The refrigerant can be selected depending on the particular application of the applicator and the desired cooling effect. An example of a suitable refrigerant is an aerosol containing butane/propane, but liquefied CO2 or any other useful refrigerant can also be used. Also propellants that have a cooling effect, such as dimethyl ether, are considered to be a refrigerant.

The active component can for example comprise one or more cosmetic agents and/or one or more pharmaceutical agents and/or one or more therapeutic agents and/or one or more homoeopathic ingredients and/or one or more excipients and/or one or more further functional components, or mixtures thereof.

The invention is further explained with reference to the drawings, showing an exemplary embodiment.
- Figure 1:: shows an exemplary embodiment of an applicator on an aerosol container;
- Figure 2:: shows the applicator and aerosol container of Figure 1 in cross section along line A - A;
- Figure 3:: shows in cross section the applicator in a first position of the stop;
- Figure 4:: shows in cross section the applicator in a second position of the stop;
- Figure 5:: shows in side view an assembly of the cooling element, the shell and the seat of the embodiment of Figure 2;
- Figure 6:: shows the assembly of Figure 5 without the cooling element in perspective view;
- Figure 7:: shows the bottom side of the shell of the assembly in Figure 5;
- Figure 8:: shows the seat of the assembly of Figure 5 in perspective view.

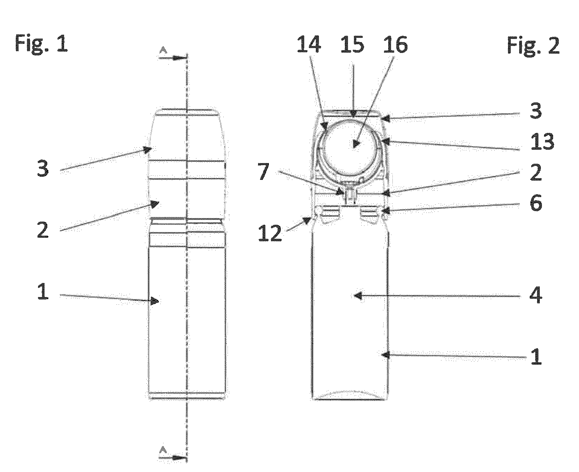

Figure 1 shows an aerosol container 1 capped with a roller ball applicator 2, which is in turn covered by a cap 3. Figure 2 shows the aerosol container 1, applicator 2 and cap 3 in cross section along line A-A of Figure 1. The aerosol container 1 has a cylindrical body 4 with a top edge formed by a conventional collar 6 and a conventional resiliently depressible nozzle 7 centrally at the containers' top. When pressed down the nozzle 7 releases the pressurized contents of the container. The pressurized contents include a liquefied refrigerant, which expands and evaporates upon release from the container. The refrigerant may be mixed with one or more pharmaceutically and/or cosmetically active compounds and/or other functional components.

The roller ball applicator 2 comprises a cylindrical casing 11 with a lower edge 12 profiled to form a tight snap joint with the collar 6 of the aerosol container 4. The upper edge 13 of the cylindrical casing 11 narrows down to a central opening 14 exposing a contact surface 15 of a spherical steel cooling element 16.

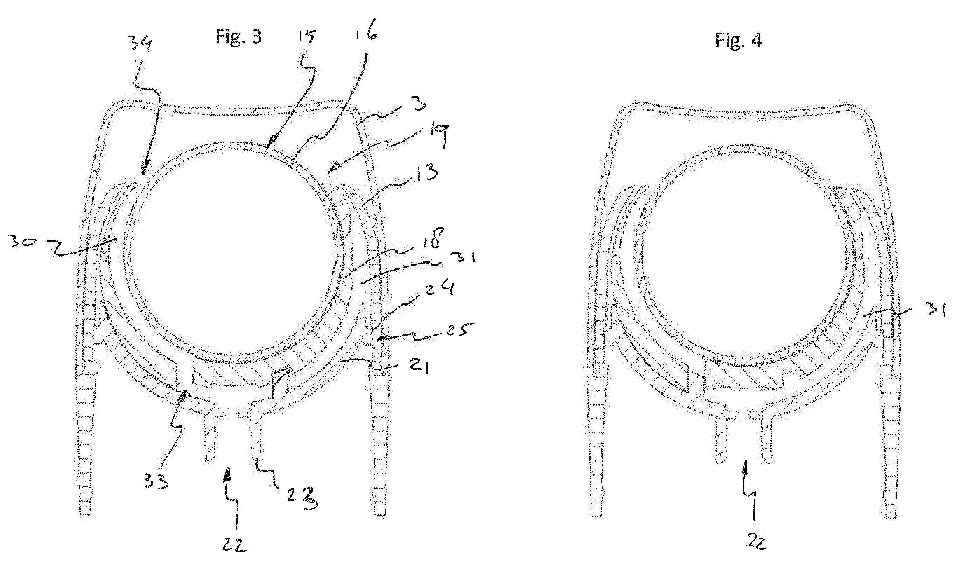

The applicator 2 is shown in more detail in Figures 3 and 4. The spherical cooling element 16 is held is a spherical shell 18 with an open top end 19 bordered by a top edge adjacent the top edge of the cylindrical casing. The spherical shell 18 is held in a semi-spherical seat 21.

The semi-spherical seat 21 has a central passage 22 with a downwardly extending tubular extension 23 fitting over the nozzle 7 of the aerosol container 4. At its outer surface near its top edge the semi-spherical seat 21 is provided with an annular rim 24. This rim 24 is received in an annular recess 25 at the inner surface of the cylindrical casing 11. The recess 25 is wider than the width of the rim 24. This allows movement of the semi-spherical seat 21 in a longitudinal direction of the cylindrical casing 11.

When a user massages his skin with the applicator 2, a pressure is exerted onto the cooling element 16. The cooling element 16 is pushed inwardly together with the shell 18 and the seat 21, which pushes down and actuates the nozzle 7 of the aerosol container 1. This movement of the seat 21 is limited by the width of the annular recess 25 receiving the annular rim 24.

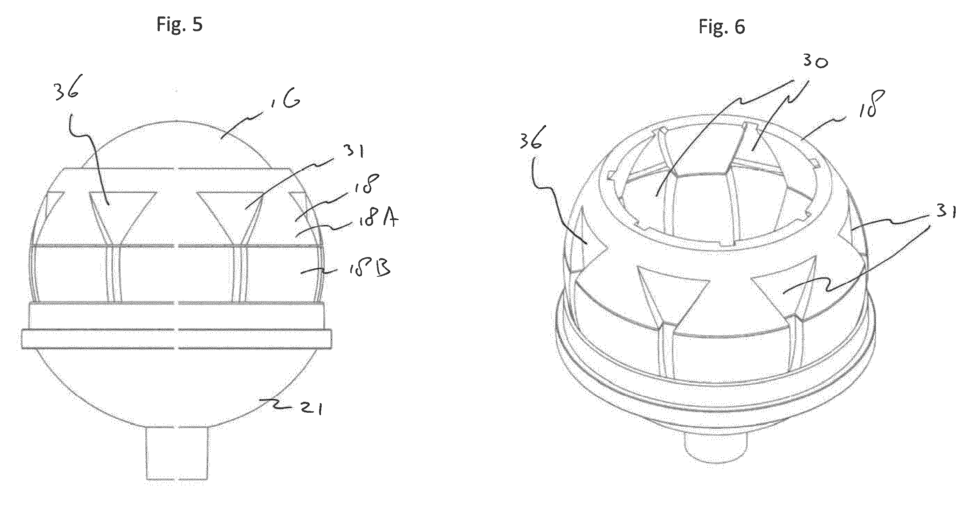

The assembly of the cooling element 16, shell 18 and seat 21 is shown in side view in Figure 5. Figure 6 shows the assembly without the cooling element 16 to show the interior side of the shell 18. The shell 18 is provided with a series of direct heat exchange channels 30 at its inner side and a series of indirect heat exchange channels 31 at its outer side.

Figure 3 shows how each direct heat exchange channel 30 extends between an inlet 33 near the passage 22 through the seat 21, and an outlet 34 at the contact face 15 of the spherical cooling element 16. The inlet 33 crosses the full wall thickness of the shell 18, so the direct heat exchange channels 30 are directly bordered by the spherical cooling element 16.

Each indirect heat exchange channel 31 is formed by a recess in the outer surface of the shell 18. A bottom side of the channel 31 forms a partition 32 separating the channel 31 from the cooling element 16. The partition 32 has a lower heat conductivity than the material of the cooling element 16. The channels 31 run from the passage 22 in the seat 21 to a point just below the upper edge of the shell 18. At this point the channel 31 has an outlet section 36 that gradually widens and gradually becomes less deep relative to the outer surface of the shell 18 (see Figures 5 and 6). Due to this outlet configuration dispensed refrigerant and active ingredient are distributed along the edges of the contact face 15.

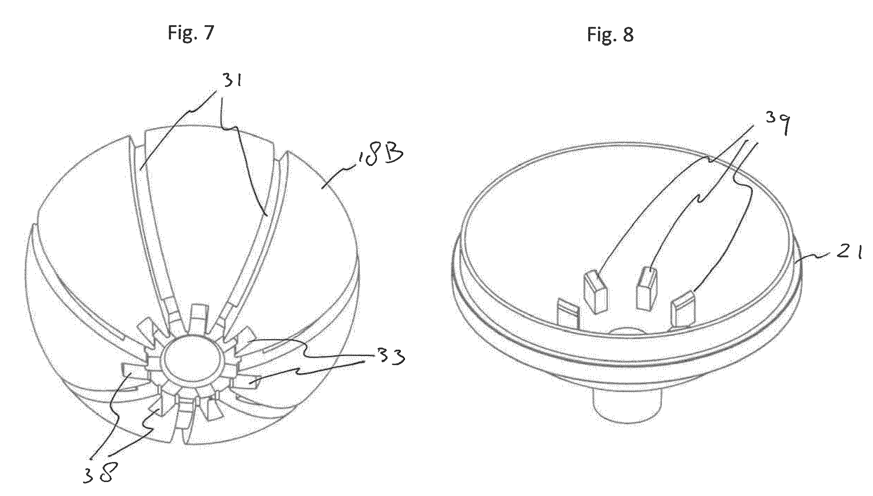

For easy moulding the shell 18 is made of an annular upper part 18A and a semi-spherical lower part 18B. The lower part 18B is shown separately in Figure 7. The bottom is a closed, shallow recess (not an opening), surrounded by a circular array of the inlets 33 leading to the channels 30 at the interior of the shell 18. The channels 31 at the exterior of the shell 18 are provided with blind recesses 38 at the same radial distance as the inlets 33, relative to a center line of the shell 18. The recesses 38 and the inlets 33 have the same width and length.

The concave side of the seat 21 is provided with a circular array of teeth or stops 39 having the same radial distance as the recesses 38 and the inlets 33, relative to a centerline of the applicator 2. The number of stops 39 is equal to the number of inlets 33 and also equal to the number of recesses 38. The stops 39 are configured to fit in the inlets 33 and in the recesses 38.

The shell 18 can be put into the seat 21 in a position where the stops 39 fit into the inlets 33 or in a position wherein the stops 39 fit into the recesses 38. When the stops 39 are fit into the inlets 33, the interior channels 30 are blocked (see Figure 4). In this position, the content of the aerosol container is dispensed via the indirect heat exchange channels 31. A passing flow of refrigerant extracts heat from the cooling element 16 indirectly via the wall 32 of the shell 18. The shell 18 has a substantially lower heat conductivity than the cooling element 16, so the cooling effect will be moderate.

If the stops 39 are fit into the recesses 38, the indirect heat exchange channels 31 are blocked by the stops 39 and the direct heat exchange channels 30 are opened. In this position, the content of the aerosol container 1 is dispensed via the direct heat exchange channels 30 so the flow of refrigerant contacts the cooling element 16 directly and heat is extracted directly from the cooling element 16. The cooling effect will be maximized and will be substantially stronger than the cooling effect obtained with the stops in the first position.

The shown embodiment can be adjusted by a manufacturer to provide a refrigerant fixed flow path and cooling effect for a given product. In other embodiments, the shell and/or the seat may be provided with an actuator accessible for a user to allow adjustment of the cooling effect by the user. In such a case the inlets 33 and the recesses 38 may for example be integrated in an annular recess allowing rotational movement of the stops 39 between a position blocking the indirect heat exchange channels 31 and a position blocking the direct heat exchange channels 30.

## Claims

1. Applicator (2) comprising a cooling element (16) and at least two flow channels (30, 31) extending between respective refrigerant inlets and refrigerant outlets, the flow channels providing different thermal conductivity between the cooling element and a refrigerant passing the respective flow channel,
wherein the applicator comprises at least one stop (39) for selectively blocking one or more of the flow channels.

2. Applicator according to claim 1, wherein the cooling element (16) is rotationally mounted in a shell and projects out of the shell to present a contact surface for a user.

3. Applicator according to claim 2, wherein the cooling element (16) is spherical.

4. Applicator according to claim 2 or 3, wherein the flow channels comprise:
- at least one direct heat exchange channel (30) confined by the surface of the cooling element;
- at least one indirect heat exchange channel (31) separated from the surface of the cooling element by a partition (32).

5. Applicator according to claim 4, further comprising at least one supply channel (22) communicating with a reservoir (1) for a refrigerant;
wherein the one or more stops (39) can selectively be positioned into a first position closing the one or more direct heat exchange channels and connecting the one or more indirect heat exchange channels to the supply channel, or into a second position closing the one or more indirect heat exchange channels and connecting the one or more direct heat exchange channels to the supply channel.

6. Applicator according to claim 4 or 5, wherein the shell (18) is held in a seat, wherein the relative position of the shell and the seat (21) is adjustable.

7. Applicator according to claim 6, wherein the one or more stops (39) comprise at least one projection of the seat (21),
wherein the number of projections (39), the number of direct heat exchange channels (30) and the number of indirect heat exchange channels (31) are equal, wherein the direct and indirect heat exchange channels are each provided with a recess (33, 38) matching the projections, wherein the projections are received in respective recesses in the direct heat exchange channels in said first position, and wherein the projections are received in respective recesses in the indirect heat exchange channels in said second position.

8. Applicator, optionally according to any preceding claim,
wherein the applicator (2) comprises a single reservoir (1) for containing a mixture of a liquid refrigerant and a cosmetically and/or pharmaceutically active component.
